(19) Europäisches Patentamt
European Patent Office
Office européen des brevets

(11) Veröffentlichungsnummer: 0 374 095 A2

# (12) EUROPÄISCHE PATENTANMELDUNG

(21) Anmeldenummer: 89810919.4

(22) Anmeldetag: 05.12.89

(51) Int. Cl.⁵: C07D 211/62, C07D 211/26, C07D 211/22, A61K 31/445

Patentansprüche für folgende Vertragsstaaten: ES + GR

(30) Priorität: 12.12.88 CH 4574/88

(43) Veröffentlichungstag der Anmeldung: 20.06.90 Patentblatt 90/25

(84) Benannte Vertragsstaaten: AT BE CH DE ES FR GB GR IT LI LU NL SE

(71) Anmelder: CIBA-GEIGY AG
Klybeckstrasse 141
CH-4002 Basel(CH)

(72) Erfinder: Caravatti, Giorgio, Dr.
Kurzelängeweg 30
CH-4123 Allschwil(CH)
Erfinder: Stanek, Jaroslav, Dr.
Hangstrasse 9
CH-4144 Arlesheim(CH)
Erfinder: Frei, Jörg, Dr.
Buechring 36
CH-4434 Hölstein(CH)

## (54) Piperidinderivate.

(57) Verbindungen der Formel I

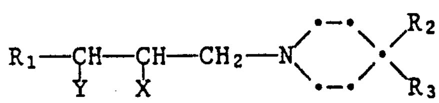

(I)

worin X, Y, $R_1$, $R_2$, und $R_3$ die in der Beschreibung angegebenen Bedeutungen haben, weisen wertvolle pharmazeutische Eigenschaften auf und sind insbesondere gegen Tumoren wirksam. Sie werden in an sich bekannter Weise hergestellt.

EP 0 374 095 A2

## Piperidinderivate

Die Erfindung betrifft Verbindungen der Formel I

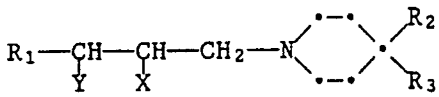

(I)

worin $R_1$ $C_1$-$C_{30}$-Alkyl bedeutet; $R_2$ Carboxy, Niederalkoxycarbonyl, Carbamoyl, N-Niederalkylcarbamoyl, N,N-Diniederalkylcarbamoyl, Cyano oder Niederalkyl, welches durch Hydroxy, Niederalkoxy, Acyloxy, Amino, Niederalkylamino, Diniederalkylamino oder Acylamino substituiert ist, bedeutet; $R_3$ für Wasserstoff, Niederalkyl oder Aryl steht; worin einer der Reste X und Y Hydroxy, Niederalkoxy oder Acyloxy bedeutet und der andere der Reste X und Y für Wasserstoff steht; oder worin beide Reste X und Y auch Wasserstoff bedeuten können, wenn $R_2$ für Cyano oder für durch Amino oder Acylamino substituiertes Niederalkyl steht und $R_1$ $C_2$-$C_{30}$-Alkyl bedeutet; und ihre Salze, Verfahren zur Herstellung dieser Verbindungen, pharmazeutische Präparate, die diese Verbindungen enthalten, die Verwendung dieser Verbindungen zur therapeutischen Behandlung des menschlichen oder tierischen Körpers oder zur Herstellung pharmazeutischer Präparate.

Die vor- und nachstehend verwendeten Allgemeinbegriffe haben im Rahmen der vorliegenden Anmeldung die folgenden Bedeutungen:

Das Präfix "Nieder" bezeichnet einen Rest mit 1 bis 7 und insbesondere mit 1 bis 4 Kohlenstoffatomen.

Niederalkyl - als solches und auch in zusammengesetzten Begriffen wie z.B. Niederalkoxycarbonyl, N-Niederalkylcarbamoyl, N,N-Diniederalkylcarbamoyl, Niederalkoxy, Niederalkylamino oder Diniederalkylamino - ist z.B. n-Propyl, Isopropyl, n-Butyl, Isobutyl, sek-Butyl, tert-Butyl, n-Pentyl, Neopentyl, n-Hexyl oder n-Heptyl, vorzugsweise Ethyl und vor allem Methyl.

$C_1$-$C_{30}$-Alkyl ist vorzugsweise lineares $C_1$-$C_{30}$-Alkyl, kann aber auch verzweigt sein, und steht z.B. für Niederalkyl wie oben definiert, oder z.B. n-Octyl, n-Nonyl, n-Decyl, n-Undecyl, n-Tridecyl, n-Pentadecyl, oder auch z.B. 2,7-Dimethyloctyl. $C_1$-$C_{30}$-Alkyl ist insbesondere $C_1$-$C_{19}$-Alkyl, vor allen Dingen $C_1$-$C_{15}$-Alkyl und in erster Linie $C_7$-$C_{15}$-Alkyl.

$C_2$-$C_{30}$-Alkyl entspricht $C_1$-$C_{30}$-Alkyl mit der Ausnahme von Methyl und ist insbesondere $C_2$-$C_{19}$-Alkyl und in erster Linie $C_7$-$C_{15}$-Alkyl.

Ein Acyloxy-Rest X oder Y ist z.B. Arylcarbonyloxy, Aryl-niederalkanoyloxy, Halogen-niederalkanoyloxy, $C_1$-$C_{30}$-Alkanoyloxy, Carbamoyloxy, N-Niederalkylcarbamoyloxy, N-Arylniederalkylcarbamoyloxy oder N-Arylcarbamoyloxy, insbesondere $C_1$-$C_{18}$-Alkanoyloxy oder N-Phenylniederalkylcarbamoyloxy, und in erster Linie $C_1$-$C_{12}$-Alkanoyloxy.

Acyloxy im allgemeinen ist z.B. Arylcarbonyloxy, Aryl-niederalkanoyloxy, Halogen-niederalkanoyloxy oder Niederalkanoyloxy, und bevorzugt Niederalkanoyloxy.

Acylamino ist z.B. Arylcarbonylamino, Aryl-niederalkanoylamino, Halogen-niederalkanoylamino oder Niederalkanoylamino, und insbesondere Niederalkanoylamino.

Aryl - als solches und auch in zusammengesetzten Begriffen wie z.B. Arylcarbonyloxy, Aryl-niederalkanoyloxy, Arylcarbonylamino oder Aryl-niederalkanoylamino - ist z.B. Phenyl oder Naphthyl, wie 1- oder 2-Naphthyl, oder substituiertes Phenyl oder Naphthyl, wie z.B. Phenyl oder Naphthyl, welche durch Niederalkyl, Halogen-niederalkyl, Hydroxy, Niederalkoxy, Niederalkanoyloxy, Halogen und/oder Nitro substituiert sind. Aryl ist bevorzugt unsubstituiertes oder wie oben angegeben substituiertes Phenyl, und insbesondere Phenyl.

Arylcarbonyloxy ($\hat{=}$ Aroyloxy) ist z.B. Benzoyloxy oder Naphthoyloxy.

Aryl-niederalkanoyloxy ist z.B. Phenylniederalkanoyloxy, wie Phenylacetyloxy oder Phenylpropionyloxy.

Halogen-niederalkanoyloxy ist z.B. Trifluoracetyloxy.

$C_1$-$C_{30}$-Alkanoyloxy ist z.B. Palmitoyloxy, Stearoyloxy oder Niederalkanoyloxy.

Niederalkanoyloxy ist z.B. Acetyloxy, Propionyloxy oder Pivaloyloxy, ferner auch z.B. Formyloxy.

Arylcarbonylamino ($\hat{=}$ Aroylamino) ist z.B. Benzoylamino oder Naphthoylamino.

Aryl-niederalkanoylamino ist z.B. Phenylniederalkanoylamino, wie Phenylacetylamino oder Phenylpropionylamino.

Halogen-niederalkanoylamino ist z.B. Trifluoracetylamino.

Niederalkanoylamino ist z.B. Acetylamino, Propionylamino oder Pivaloylamino, ferner auch z.B. Formylamino.

Halogen ist z.B. Fluor oder Jod, insbesondere Brom und vor allem Chlor.

Halogen-niederalkyl ist z.B. Trifluormethyl.

Salze von erfindungsgemässen Verbindungen sind in erster Linie pharmazeutisch verwendbare, nichttoxische Salze. Beispielsweise können Verbindungen der Formel I mit basischen Gruppen Säureadditionssalze, z.B. mit anorganischen Säuren, wie Salzsäure, Schwefelsäure oder Phosphorsäure, oder mit geeigneten organischen Carbon- oder Sulfonsäuren, z.B. Essigsäure, Fumarsäure oder Methansulfonsäure, oder z.B. mit Aminosäuren, wie Arginin oder Lysin, bilden. Bei Anwesenheit von mehreren basischen Gruppen können Mono- oder Polysalze gebildet werden. Verbindungen der Formel I mit einer sauren Gruppe, z.B. Carboxy, und einer basischen Gruppe, z.B. Amino, können z.B. in Form von inneren Salzen, d.h. in zwitterionischer Form vorliegen, oder es kann ein Teil des Moleküls als inneres Salz, und ein anderer Teil als normales Salz vorliegen.

Zur Isolierung oder Reinigung können auch pharmazeutisch ungeeignete Salze, z.B. Pikrate oder Perchlorate, Verwendung finden. Zur therapeutischen Anwendung gelangen nur die pharmazeutisch verwendbaren, nichttoxischen Salze, die deshalb bevorzugt sind.

Je nach den strukturellen Gegebenheiten können die Verbindungen der vorliegenden Erfindung in Form von Isomerengemischen oder von reinen Isomeren vorliegen.

Die erfindungsgemässen Verbindungen der Formel I weisen wertvolle, insbesondere pharmakologisch verwendbare, Eigenschaften auf. Insbesondere zeigen sie eine starke inhibierende Wirkung auf die Proteinkinase C. Die von Phospholipiden und Calcium abhängige Proteinkinase C kommt innerhalb der Zelle in mehreren Formen vor und beteiligt sich an verschiedenen fundamentalen Vorgängen, wie Signalübertragung, Proliferation und Differenzierung sowie auch der Ausschüttung von Hormonen und Neurotransmittern. Die Aktivierung dieser Enzyme erfolgt bekanntlich entweder durch eine über Rezeptoren vermittelte Hydrolyse von Phospholipiden der Zellmembran oder durch eine direkte Interaktion mit gewissen Tumorfördernden Wirkstoffen. Die rezeptorvermittelte Signalübertragung via Abbau von Inositolphospholipiden kann durch die Modulierung der Aktivität von Proteinkinase C beeinflusst werden. Substanzen, die fähig sind, die Aktivität der Proteinkinase C selektiv abzuwandeln, - wie die Verbindungen der Formel I - können als tumorhemmende, immunomodulierende, entzündungshemmende, antibakterielle und antiparasitäre, wie antitrypanozide oder Antimalaria-, Wirkstoffe Verwendung finden.

Die erfindungsgemässen Verbindungen sind ausserdem antiviral wirksam und können z.B. zur Behandlung von durch HIV-Virus induzierten Erkrankungen, wie ARC oder AIDS, von Interesse sein.

Die wertvollen pharmakologischen Eigenschaften der erfindungsgemässen Verbindungen lassen sich z.B. mit den folgenden biologischen Standard-tests nachweisen: 1. Modulierung der Wirkung von Proteinkinase C (aus Rattenhirn); 2. Antiproliferative Wirkung gegen z.B. das humane T24 Blasencarcinom in vitro; 3. Antitumorwirkung in vivo z.B. bei den humanen T24 Blasen- und MBA 9812 Lungenxenografts an der Nacktmaus; 4. Hemmung der HIV-Reverstranscriptase und DNA-Polymerase.

Die effektiven Konzentrationen der erfindungsgemässen Verbindungen, die zu einer 50%igen Hemmung in den in vitro Tests führen (IC 50-Werte), liegen minimal bei ca. 10 nM, die effektiven Dosen bei in vivo Experimenten entsprechen höchstens einem Fünftel der Dosis tolerata maxima und liegen zwischen ca. 0.5 und 50 mg/kg.

Daher sind die Verbindungen der Formel I z.B. geeignet zur Behandlung benigner und maligner Tumoren. Sie können Tumorregressionen bewirken und ferner die Verbreitung von Tumorzellen sowie das Wachstum von Mikrometastasen verhindern.

Weiterhin sind die Verbindungen der Formel I, worin $R_2$ Cyano bedeutet, auch wertvolle Zwischenprodukte zur Herstellung anderer Verbindungen der Formel I, z.B. solcher, worin $R_2$ Aminomethyl bedeutet. Letztere lassen sich aus ersteren durch Reduktion, etwa mit Lithiumaluminiumhydrid und Aluminiumtrichlorid, herstellen.

Bevorzugt sind die Verbindungen der Formel I, worin $R_1$ $C_1$-$C_{19}$-Alkyl bedeutet; $R_2$ Carboxy, Niederalkoxycarbonyl, Carbamoyl, N-Niederalkylcarbamoyl, N,N-Diniederalkylcarbamoyl, Cyano oder Niederalkyl, welches durch Hydroxy, Niederalkoxy, Niederalkanoyloxy, Amino, Niederalkylamino, Diniederalkylamino oder Niederalkanoylamino substituiert ist, bedeutet; $R_3$ für Wasserstoff, Niederalkyl oder Phenyl steht; worin einer der Reste X und Y Hydroxy, Niederalkoxy, $C_1$-$C_{18}$-Alkanoyloxy, Carbamoyloxy, N-Niederalkylcarbamoyloxy, N-Phenylniederalkylcarbamoyloxy oder N-Phenylcarbamoyloxy bedeutet und der andere der Reste X und Y für Wasserstoff steht, oder worin beide Reste X und Y auch Wasserstoff bedeuten können, wenn $R_2$ für Cyano oder für durch Amino oder Niederalkanoylamino substituiertes Niederalkyl steht und $R_1$ $C_2$-$C_{19}$-Alkyl bedeutet; wobei Phenylreste unsubstituiert oder durch Niederalkyl, Halogen-niederalkyl, Hydroxy, Niederalkoxy, Niederalkanoyloxy, Halogen und/oder Nitro substituiert sind; und ihre Salze.

Besonders bevorzugt sind die Verbindungen der Formel I, worin $R_1$ $C_1$-$C_{15}$-Alkyl bedeutet; $R_2$ Carboxy, Niederalkoxycarbonyl, Carbamoyl, Cyano oder Niederalkyl, welches durch Hydroxy, Amino oder Niederalka-

noylamino substituiert ist, bedeutet; $R_3$ für Wasserstoff, Niederalkyl oder Phenyl steht; worin einer der Reste X und Y Hydroxy, $C_1$-$C_{12}$-Alkanoyloxy oder N-Phenylniederalkylcarbamoyloxy bedeutet und der andere der Reste X und Y für Wasserstoff steht, oder worin beide Reste X und Y auch Wasserstoff bedeuten können, wenn $R_2$ für Aminomethyl steht und $R_1$ $C_2$-$C_{15}$-Alkyl bedeutet; und ihre Salze.

In erster Linie bevorzugt sind die Verbindungen der Formel I, worin $R_1$ $C_7$-$C_{15}$-Alkyl bedeutet; $R_2$ Carboxy, Niederalkoxycarbonyl, Carbamoyl, Hydroxymethyl, Aminomethyl oder Niederalkanoylaminomethyl bedeutet; $R_3$ für Wasserstoff, Methyl oder Phenyl steht; worin einer der Reste X und Y Hydroxy bedeutet und der andere der Reste X und Y für Wasserstoff steht, oder worin beide Reste X und Y auch Wasserstoff bedeuten können, wenn $R_2$ für Aminomethyl steht; und ihre Salze.

Vor allen Dingen bevorzugt sind die Verbindungen der Formel I, worin $R_1$ $C_7$-$C_{15}$-Alkyl bedeutet, $R_2$ für Aminomethyl steht, $R_3$ Phenyl bedeutet, und (a) X Hydroxy bedeutet und Y für Wasserstoff steht, oder (b) X Wasserstoff bedeutet und Y für Hydroxy steht, oder (c) X und Y Wasserstoff bedeuten; und ihre Salze.

Die Erfindung betrifft vor allem die in den Beispielen beschriebenen spezifischen Verbindungen und pharmazeutisch anwendbare Salze davon.

Die Verbindungen der Formel I können in an sich bekannter Weise hergestellt werden, indem man z.B.

(a) eine Verbindung der Formel II

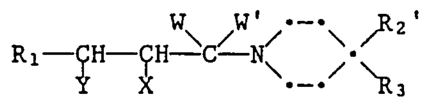

(II)

worin $R_1$, $R_3$, X und Y die unter Formel I angegebene Bedeutung haben, $R_2'$ eine Gruppe, die durch Reduktion in einen Rest $R_2$ überführbar ist, bedeutet, oder, falls W und W′ zusammen Oxo bedeuten, auch für einen Rest $R_2$ wie unter Formel I definiert stehen kann, und W und W′ zusammen eine Oxogruppe bedeuten oder für zwei Wasserstoffatome stehen, reduziert, oder

(b) eine Verbindung der Formel III

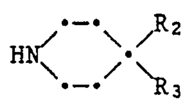

(III)

worin $R_2$ und $R_3$ die unter Formel I angegebene Bedeutung haben, mit einer Verbindung der Formel IV

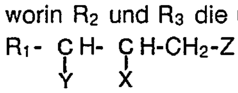

(IV)

worin $R_1$, X und Y die unter Formel I angegebene Bedeutung haben und Z eine nukleofuge Abgangsgruppe ist und worin X und Z zusammen auch eine Epoxygruppe bedeuten können, alkyliert, oder

(c) zur Herstellung von Verbindungen der Formel I, worin X oder Y Hydroxy bedeutet, in einer Verbindung der Formel V

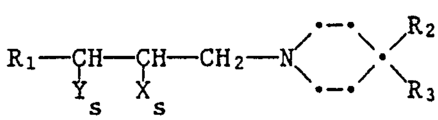

(V)

worin $R_1$, $R_2$ und $R_3$ die unter Formel I angegebene Bedeutung haben, einer der Reste $X_s$ und $Y_s$ eine durch eine Schutzgruppe geschützte Hydroxygruppe bedeutet und der andere der Reste $X_s$ und $Y_s$ für Wasserstoff steht, die Hydroxy-Schutzgruppe abspaltet; und/oder, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder ein erhaltenes Gemisch von isomeren Verbindungen der Formel I in die einzelnen Isomeren auftrennt.

In der folgenden näheren Beschreibung der Verfahren a)-c) haben die Symbole $R_1$, $R_2$, $R_3$, X und Y jeweils die unter Formel I angegebene Bedeutung, sofern nichts anderes angegeben ist.

Verfahren (a):

Als Gruppen $R_2'$, die durch Reduktion in einen Rest $R_2$ überführbar sind, sind z.B. Cyano und Carbamoyl, welche unter den gewählten reduktiven Bedingungen normalerweise in Aminomethyl überführt werden, oder Niederalkoxycarbonyl und Carboxy, welche unter den gewählten reduktiven Bedingungen in Hydroxymethyl überführt werden, zu erwähnen.

Die Reduktion gemäss Verfahren (a) wird, wenn $R_2'$ in der Verbindung der Formel II Cyano bedeutet, z.B. mit einem Gemisch aus Lithiumaluminiumhydrid ($LiAlH_4$) und Aluminiumtrichlorid durchgeführt.

Verbindungen der Formel II, worin $R_2'$ Carbamoyl, Niederalkoxycarbonyl oder Carboxy bedeutet, werden z.B. mit $LiAlH_4$ allein reduziert.

Die Ausgangsverbindungen der Formel II, worin W und W' zusammen Oxo (=O) bedeuten, werden z.B. dadurch hergestellt, dass man eine Verbindung analog zur Formel III, die anstelle des Restes $R_2$ eine Gruppe $R_2'$ wie unter Formel II definiert enthält, mit einer Säure der Formel $R_1$-CHY-CHX-COOH, oder einem reaktionsfähigen Derivat davon, etwa einem Säurehalogenid oder -anhydrid, acyliert. Die Ausgangsverbindungen der Formel II, worin W und W' für zwei Wasserstoffatome stehen, entsprechen in der Regel Verbindungen der Formel I und werden z.B. gemäss Verfahren (b) oder (c) hergestellt.

Verfahren (b):

Die Umsetzung gemäss dem Verfahren (b) verläuft im Sinne einer konventionellen nukleophilen Substitutionsreaktion. Dabei wird das sekundäre Stickstoffatom in einer Verbindung der Formel III alkyliert.

Gegebenenfalls wird die Reaktion in Gegenwart von anorganischen oder organischen säurebindenden Mitteln, z.B. tertiären Aminen, etwa Triethylamin, oder Kaliumcarbonat, durchgeführt.

In einer Verbindung der Formel IV steht eine nukleofuge Abgangsgruppe Z z.B. für Halogen, insbesondere Chlor, Brom oder Iod, oder aliphatisch-oder aromatisch-substituiertes Sulfonyloxy, z.B. Methylsulfonyloxy oder 4-Methylphenylsulfonyloxy.

Wenn X und Z zusammen in einer Verbindung der Formel IV eine Epoxygruppe

( 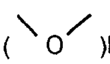 )bedeuten, so werden bei Durchführung des Verfahrens (b) Verbindungen der Formel I erhalten, worin X Hydroxy bedeutet.

Die Ausgangsverbindungen der Formel III und IV sind an sich bekannt oder können analog zu bekannten Verbindungen hergestellt werden.

Verfahren (c):

Eine durch eine Schutzgruppe geschützte Hydroxygruppe $X_s$ bzw. $Y_s$ ist z.B. organisches Silyloxy, insbesondere Triniederalkylsilyloxy, z.B. Trimethylsilyloxy, Dimethyl-tert-butylsilyl und vor allem Dimethylthexylsilyloxy (thexyl $\hat{=}$ 2,3-dimethyl-2-butyl), oder Acyloxy, insbesondere Niederalkanoyloxy, z.B. Formyloxy, Acetyloxy oder Pivaloyloxy; Halogenniederalkanoyloxy, z.B. Chloracetyloxy, Dichloracetyloxy, Trichloracetyloxy oder Trifluoracetyloxy; Niederalkoxy-niederalkanoyloxy, z.B. Methoxyacetyloxy; Arylniederalkoxyniederalkanoyloxy, z.B. Triphenylmethoxyacetyloxy; Aryloxy-niederalkanoyloxy, z.B. Phenoxyacetyloxy; Benzoyloxy; Niederalkoxycarbonyloxy, z.B. Methoxycarbonyloxy, Ethoxycarbonyloxy oder tert-Butyloxycarbonyloxy; oder Arylniederalkoxycarbonyloxy, z.B. jeweils unsubstituiertes oder durch Nitro substituiertes Benzyloxycarbonyloxy oder Diphenylmethoxycarbonyloxy.

Die Abspaltung der Hydroxyschutzgruppe gemäss Verfahren (c) erfolgt in an sich bekannter Weise durch Solvolyse, insbesondere Hydrolyse, oder auch Alkoholyse. Organisches Silyl wird z.B. durch Behandlung mit Wasser, Salzsäure oder Methanol abgespalten. Bevorzugte Systeme zur Abspaltung von organischem Silyl sind z.B. (a) Essigsäure/Tetrahydrofuran/Wasser, (b) 1 % konz. Salzsäure in Ethanol, (c) Fluorwasserstoff-Harnstoff- Komplex in Cyclohexan, (d) Tetrabutylammoniumfluorid in Tetrahydrofuran und (e) Trifluoressigsäure/Acetonitril/Wasser. Acylgruppen werden insbesondere durch Alkoholyse, aber auch Hydrolyse, in Gegenwart einer Base, z.B. Natrium- oder Kaliumethoxid oder Natrium- oder Kaliumhydroxid abgespalten.

Die Ausgangsverbindungen der Formel V werden z.B. dadurch hergestellt, dass man eines der Verfahren (a) und (b) zur Herstellung von Verbindungen der Formel I bzw. eine Umwandlung einer Verbindung der Formel I in eine andere Verbindung der Formel I mit geschützter Hydroxygruppe X bzw. Y durchführt.

Verbindungen der Formel I können in andere Verbindungen der Formel I umgewandelt werden.

Zum Beispiel können Verbindungen der Formel I, worin $R_2$ Cyano bedeutet, wie im Verfahren (a)

beschrieben durch Reduktion, etwa mit einem Gemisch aus $LiAlH_4$ und $AlCl_3$, in Verbindungen der Formel I, worin $R_2$ Aminomethyl bedeutet, überführt werden.

Ferner können Verbindungen der Formel I, worin $R_2$ Carbamoyl bzw. Niederalkoxycarbonyl oder Carboxy bedeutet, wie im Verfahren (a) beschrieben durch Reduktion, z.B. mit $LiAlH_4$, in Verbindungen der Formel I, worin $R_2$ Aminomethyl bzw. Hydroxymethyl bedeutet, überführt werden.

Verbindungen der Formel I, worin $R_2$ Niederalkoxycarbonyl bedeutet, können durch Hydrolyse, z.B. im basischen Medium, etwa mit NaOH oder KOH in Niederalkanolen, in Verbindungen der Formel I, worin $R_2$ Carboxy bedeutet, überführt werden.

Verbindungen der Formel I, worin X oder Y Hydroxy bedeuten, können durch Umsetzung mit Niederalkylierungs- bzw. Acylierungsmitteln in Verbindungen der Formel I überführt werden, worin X oder Y Niederalkoxy bzw. Acyloxy bedeuten. Geeignete Niederalkylierungsmittel sind z.B. Niederalkylhalogenide, -mesylate oder -tosylate, die vorzugsweise in Gegenwart von starken Basen, z.B. Natriumhydrid, angewendet werden. Als Acylierungs mittel kommen z.B. Alkancarbonsäuren, insbesondere reaktionsfähige Derivate davon, wie Säurechloride oder -anhydride, in Frage, die vorzugsweise in Gegenwart von anorganischen oder organischen säurebindenden Mitteln angewendet werden. Führt man die Acylierung der OH-Gruppe X bzw. Y mit z.B. Niederalkyl-, Phenylniederalkyl- oder Phenylisocyanaten durch, so erhält man Verbindungen der Formel I, worin X bzw. Y N-Niederalkylcarbamoyloxy, N-Phenylniederalkylcarbamoyloxy oder N-Phenylcarbamoyloxy bedeutet.

Umgekehrt lassen sich die Verbindungen der Formel I, worin X bzw. Y Carbamoyloxy, N-Niederalkylcarbamoyloxy, N-Phenylniederalkylcarbamoyloxy oder N-Phenylcarbamoyloxy bedeutet, z.B. durch Hydrolyse mittels Natronlauge oder durch Reduktion mit $LiAlH_4$ wieder in die analogen Verbindungen der Formel I überführen, worin X bzw. Y Hydroxy bedeutet.

Acyliert man die OH-Gruppe X bzw. Y in einer Verbindung der Formel I mit einem optisch aktiven Isocyanat, z.B. (-)-1-Phenylethylisocyanat, so erhält man ein Diastereomerengemisch von Urethanen. Die einzelnen Diastereomeren lassen sich auftrennen und z.B. durch Hydrolyse mit Natronlauge oder durch Reduktion mit $LiAlH_4$ in die reinen Enantiomeren der Verbindungen der Formel I, worin X bzw. Y Hydroxy bedeutet, überführen.

Verfahrensgemäss erhältliche freie Verbindungen der Formel I mit salzbildenden Eigenschaften können in an sich bekannter Weise in ihre Salze übergeführt werden, Verbindungen mit basischen Eigenschaften durch Behandeln mit Säuren oder geeigneten Derivaten davon, Verbindungen mit sauren Eigenschaften durch Behandeln mit Basen oder geeigneten Derivaten davon.

Erfindungsgemäss erhältliche Gemische von Isomeren können in an sich bekannter Weise in die einzelnen Isomeren aufgetrennt werden, Racemate z.B. durch Bilden von Salzen mit optisch reinen salzbildenden Reagentien und Auftrennen des so erhältlichen Diastereomerengemisches, z.B. mittels fraktionierter Kristallisation.

Setzt man bei den Verfahren (a), (b) und (c) zur Herstellung von Verbindungen der Formel I optisch aktive Ausgangsverbindungen ein, insbesondere optisch aktive Epoxide der Formel IV beim Verfahren (b), so erhält man die einzelnen Isomeren direkt bei der Durchführung der entsprechenden Verfahren.

Die oben angeführten Reaktionen können unter an sich bekannten Reaktionsbedingungen durchgeführt werden, in Ab- oder üblicherweise Anwesenheit von Lösungs- oder Verdünnungsmitteln, vorzugsweise solchen, die gegenüber den verwendeten Reagenzien inert sind und diese lösen, in Ab-oder Anwesenheit von Katalysatoren, Kondensationsmitteln oder neutralisierenden Agentien, je nach Art der Reaktion und/oder der Reaktionsteilnehmer bei erniedrigter, normaler oder erhöhter Temperatur, z.B. im Temperaturbereich von etwa -70° C bis etwa 190° C, vorzugsweise von etwa -20° C bis etwa 150° C, z.B. beim Siedepunkt des verwendeten Lösungsmittels, unter atmosphärischem Druck oder in einem geschlossenen Gefäss, gegebenenfalls unter Druck, und/oder in einer inerten Atmosphäre, z.B. unter einer Stickstoffatmosphäre.

Im Verfahren der vorliegenden Erfindung werden vorzugsweise solche Ausgangsstoffe eingesetzt, die zu den eingangs als besonders wertvoll beschriebenen Verbindungen führen.

Die Erfindung betrifft auch diejenigen Ausführungsformen des Verfahrens, bei denen man von einer auf einer beliebigen Verfahrensstufe als Zwischenprodukt erhältlichen Verbindung ausgeht und die fehlenden Verfahrensschritte durchführt, oder bei denen ein Ausgangsstoff unter den Reaktionsbedingungen gebildet oder in Form eines Derivates, z.B. eines Salzes davon, verwendet wird.

Die vorliegende Erfindung betrifft ebenfalls pharmazeutische Präparate, die als Wirkstoff eine der pharmakologisch wirksamen Verbindungen der Formel I enthalten. Besonders bevorzugt sind Präparate zur enteralen, insbesondere oralen, sowie zur parenteralen Verabreichung. Die Präparate enthalten den Wirkstoff allein oder vorzugsweise zusammen mit einem pharmazeutisch anwendbaren Trägermaterial. Die Dosierung des Wirkstoffs hängt von der zu behandelnden Krankheit, sowie von Spezies, deren Alter, Gewicht und individuellem Zustand, sowie von der Applikationsweise ab.

Die pharmazeutischen Präparate enthalten von etwa 5 % bis etwa 95 % des Wirkstoffs, wobei einzeldosierte Applikationsformen vorzugsweise von etwa 20 % bis etwa 90 % und nicht-einzeldosierte Applikationsformen vorzugsweise etwa 5 % bis etwa 20 % Wirkstoff aufweisen. Dosiseinheitsformen, wie Dragées, Tabletten oder Kapseln, enthalten von etwa 0,01 g bis etwa 1,0 g des Wirkstoffs.

Die pharmazeutischen Präparate der vorliegenden Erfindung werden in an sich bekannter Weise, z.B. mittels konventioneller Misch-, Granulier-, Dragier-, Lösungs-, oder Lyophilisierungsverfahren hergestellt. So kann man pharmazeutische Zusammensetzungen zur oralen Anwendung erhalten, indem man den Wirkstoff mit einem oder mehreren festen Trägerstoffen kombiniert, ein erhaltenes Gemisch gegebenenfalls granuliert, und das Gemisch bzw. Granulat, wenn erwünscht, gegebenenfalls durch Zugabe von zusätzlichen Hilfsstoffen, zu Tabletten oder Dragées-Kernen verarbeitet.

Geeignete Trägerstoffe sind insbesondere Füllstoffe, wie Zucker, z.B. Lactose, Saccharose, Mannit oder Sorbit, Cellulosepräparate und/oder Calciumphosphate, z.B. Tricalciumphosphat oder Calciumhydrogenphosphat, ferner Bindemittel, wie Stärken, z.B. Mais-, Weizen-, Reis- oder Kartoffelstärke, Methylcellulose, Hydroxypropylmethylcellulose, Natriumcarboxymethylcellulose und/oder Polyvinylpyrrolidon, und/oder, wenn erwünscht, Sprengmittel, wie die obgenannten Stärken, ferner Carboxymethylstärke, quervernetztes Polyvinylpyrrolidon, Alginsäure oder ein Salz davon, wie Natriumalginat. Zusätzliche Hilfsmittel sind in erster Linie Fliessregulier- und Schmiermittel, z.B. Kieselsäure, Talk, Stearinsäure oder Salze davon, wie Magnesium- oder Calciumstearat, und/oder Polyethylenglykol, oder Derivate davon.

Dragées-Kerne können mit geeigneten, gegebenenfalls Magensaft-resistenten Ueberzügen versehen werden, wobei man u.a. konzentrierte Zuckerlösungen, welche gegebenenfalls arabischen Gummi, Talk, Polyvinyl pyrrolidon, Polyethylenglykol und/oder Titandioxid enthalten, Lacklösungen in geeigneten organischen Lösungsmitteln oder Lösungsmittelgemischen oder, zur Herstellung von Magensaft-resistenten Ueberzügen, Lösungen von geeigneten Cellulosepräparaten, wie Acetylcellulosephthalat oder Hydroxypropylmethylcellulosephthalat, verwendet. Den Tabletten oder Dragée-Ueberzügen können Farbstoffe oder Pigmente, z.B. zur Identifizierung oder zur Kennzeichnung verschiedener Wirkstoffdosen, beigefügt werden.

Oral anwendbare pharmazeutische Zusammensetzungen sind ebenfalls Steckkapseln aus Gelatine, sowie weiche, geschlossene Kapseln aus Gelatine und einem Weichmacher, wie Glycerin oder Sorbit. Die Steckkapseln können den Wirkstoff in Form eines Granulats, z.B. im Gemisch mit Füllstoffen, wie Maisstärke, Bindemitteln und/oder Gleitmitteln, wie Talk oder Magnesiumstearat, und gegebenenfalls von Stabilisatoren, enthalten. In weichen Kapseln ist der Wirkstoff vorzugsweise in geeigneten flüssigen Hilfsstoffen, wie fetten Oelen, Paraffinöl oder flüssigen Polyethylenglykolen, gelöst oder suspendiert, wobei ebenfalls Stabilisatoren zugefügt sein können.

Weitere orale Applikationsformen sind z.B. in üblicher Weise bereitete Sirups, die den Wirkstoff z.B. in suspendierter Form und in einer Konzentration von ca. 5 % bis 20 %, vorzugsweise ca. 10 % oder in einer ähnlichen Konzentration, die z.B. beim Abmessen von 5 oder 10 ml eine geeignete Einzeldosis ergibt, enthalten. Ferner kommen z.B. auch pulverförmige oder flüssige Konzentrate zur Bereitung von Shakes, z.B. in Milch, in Betracht. Solche Konzentrate können auch in Einzeldosismengen abgepackt sein.

Als rektal anwendbare pharmazeutische Präparate kommen z.B. Suppositorien in Betracht, welche aus einer Kombination des Wirkstoffs mit einer Suppositoriengrundmasse bestehen. Als Suppositoriengrundmasse eignen sich z.B. natürliche oder synthetische Triglyceride, Paraffinkohlenwasserstoffe, Polyethylenglykole oder höhere Alkanole.

Zu parenteralen Verabreichung eignen sich in erster Linie wässrige Lösungen eines Wirkstoffes in wasserlöslicher Form, z.B. eines wasserlöslichen Salzes, oder wässrige Injektionssuspensionen, welche viskositätserhöhende Stoffe, z.B. Natriumcarboxymethylcellulose, Sorbit und/oder Dextran und gegebenenfalls Stabilisatoren enthalten. Dabei kann der Wirkstoff, gegebenenfalls zusammen mit Hilfsstoffen, auch in Form eines Lyophilisats vorliegen und vor der parenteralen Verabreichung durch Zugabe von geeigneten Lösungsmitteln in Lösung gebracht werden.

Lösungen, wie sie z.B. für die parenterale Verabreichung verwendet werden, können auch als Infusionslösungen angewandt werden.

Die Erfindung betrifft ebenfalls ein Verfahren zur Behandlung der oben genannten Krankheitszustände. Die Verbindungen der vorliegenden Erfindung können prophylaktisch oder therapeutisch verabreicht werden, wobei man sie vorzugsweise in Form von pharmazeutischen Präparaten verwendet. Dabei wird bei einem Körpergewicht von etwa 70 kg eine tägliche Dosis von etwa 0,05 g bis etwa 5 g, vorzugsweise von etwa 0,1 g bis etwa 1 g einer Verbindung der vorliegenden Erfindung verabreicht.

Die nachfolgenden Beispiele illustrieren die vorliegende Erfindung; Temperaturen werden in Grad Celsius angegeben. Folgende Abkürzungen werden verwendet: THF $\hat{=}$ Tetrahydrofuran; Essigester $\hat{=}$ Essigsäureethylester; Ether $\hat{=}$ Diethylether; abs. $\hat{=}$ absolut(er, en, em); konz. $\hat{=}$ konzentriert(er, en, em).

Beispiel 1: (a) 4-Cyano-1-(2′-hydroxy-1′-decyl)-4-phenylpiperidin

18.72 g (0.12 Mol) 1,2-Epoxydecan (Aldrich) werden in 180 ml Ethanol gelöst, mit 26.72 g (0.12 Mol) 4-Cyano-4-phenylpiperidin-hydrochlorid (Aldrich) und 16.56 g (0.12 Mol) Kaliumcarbonat versetzt und 6 h unter Rückfluss erhitzt. Die anorganischen Salze werden nach dem Abkühlen abfiltriert, das Filtrat eingedampft, der Rückstand in 100 ml Methylenchlorid gelöst und mit gesättigter Natriumbicarbonat- und gesättigter Natriumchloridlösung gewaschen. Danach trocknet man die organische Phase über Magnesiumsulfat und dampft ein. Das resultierende Oel wird mit Petrolether verrieben und das kristallisierende Produkt abfiltriert, mit kaltem Petrolether gewaschen und getrocknet; Smp. 77-79°.

Analog Beispiel 1a werden die folgenden Verbindungen hergestellt:

(b) 4-Cyano-1-(2′-hydroxy-1′-hexyl)-4-phenylpiperidin, Smp. 55-58°.

(c) 4-Cyano-1-(2′-hydroxy-1′-octyl)-4-phenylpiperidin, Smp. 61-63°.

(d) 4-Cyano-1-(2′-hydroxy-1′-dodecyl)-4-phenylpiperidin, Smp. 54-56°.

(e) 4-Cyano-1-(2′-hydroxy-1′-tetradecyl)-4-phenylpiperidin.

50,4 g (0,24 Mol) 1,2-Epoxytetradecan (Aldrich) werden in 300 ml Ethanol gelöst, mit 54,4 g (0,24 Mol) 4-Cyano-4-phenylpiperidin-hydrochlorid (Aldrich) und 33,2 g (0,24 Mol) Kaliumcarbonat versetzt und 5 Stunden unter Rückfluss erhitzt. Die anorganischen Salze werden nach dem Abkühlen abfiltriert, das Filtrat eingedampft, der Rückstand in 300 ml Ether gelöst und mit 100 ml Wasser und 50 ml gesättigter Natriumchloridlösung gewaschen. Danach trocknet man die organische Phase über Kaliumcarbonat und engt bis zur Hälfte des Volumens ein. Nach Zugabe von 100 ml Petrolether wird bei 0° stehengelassen. Die Titelverbindung kristallisiert dabei aus, wird abfiltriert, mit kaltem Petrolether gewaschen und am Hochvakuum getrocknet. Smp. 68-69°.

(f) R-(-)-4-Cyano-1-(2′-hydroxy-1′-tetradecyl)-4-phenylpiperidin, Smp. 69-70°.

(g) S-(+)-4-Cyano-1-(2′-hydroxy-1′-tetradecyl)-4-phenylpiperidin, IR (KBr): 2940, 2870, 2247 (schwach), 1550, 1468 $cm^{-1}$.

(h) 4-Cyano-1-(2′-hydroxy-1′-hexadecyl)-4-phenylpiperidin, Smp. 67-69°.

(i) 4-Cyano-1-(2′-hydroxy-1′-octadecyl)-4-phenylpiperidin, Smp. 88-90°.

(j) 4-Cyano-1-(2′-hydroxy-1′-butyl)-4-phenylpiperidin, Smp. 73°.

Beispiel 2: (a) 4-Aminomethyl-1-(2′-hydroxy-1′-decyl)-4-phenylpiperidin-dihydrochlorid

Eine Lösung von 23.8 g (0.179 Mol) Aluminiumtrichlorid in 360 ml abs. THF wird unter Stickstoff und bei Raumtemperatur in eine Suspension von 6.83 g (0.179 Mol) Lithiumaluminiumhydrid in 450 ml abs. THF getropft. Es wird 15 min gerührt und dann auf 35° erwärmt. Zu dieser Mischung tropft man eine Lösung von 26.1 g (0.179 Mol) 4-Cyano-1-(2′-hydroxy-1′-decyl)-4-phenylpiperidin (s. Beispiel 1a) in 450 ml abs. THF zu. Das Reaktions gemisch wird anschliessend 2.5 h bei 40 bis 50° gerührt. Nach dem Abkühlen auf Raumtemperatur setzt man vorsichtig ca. 250 ml konzentrierte Natronlauge zu, bis das Gemisch alkalisch reagiert. Die Emulsion wird durch Kieselgur (Hyflo Super Cel, Fluka) filtriert, das Filtrat mit Ether extrahiert und die organische Phase über Natriumsulfat getrocknet und eingedampft. Der resultierende Rückstand wird in wenig Ethanol gelöst und mit 51 ml einer 10 %igen ethanolischen Salzsäurelösung versetzt. Die Titelverbindung kristallisiert dabei in Nadeln aus; Smp. 259-261°.

Analog Beispiel 2a werden die folgenden Verbindungen hergestellt:

(b) 4-Aminomethyl-1-(2′-hydroxy-1′-hexyl)-4-phenylpiperidin-dihydrochlorid, Smp. 254-256°.

(c) 4-Aminomethyl-1-(2′-hydroxy-1′-octyl)-4-phenylpiperidin-dihydrochlorid, Smp. 243-245°.

(d) 4-Aminomethyl-1-(2′-hydroxy-1′-dodecyl)-4-phenylpiperidin-dihydrochlorid, Smp. 250-253°.

(e) 4-Aminomethyl-1-(2′-hydroxy-1′-tetradecyl)-4-phenylpiperidin-dihydrochlorid.

Eine Lösung von 10,6 g (0,08 Mol) Aluminiumtrichlorid in 160 ml absolutem Tetrahydrofuran wird unter Stickstoff und bei Raumtemperatur in eine Suspension von 3,0 g (0,08 Mol) Lithiumaluminiumhydrid in 200 ml absolutem Tetrahydrofuran getropft. Es wird 15 Minuten gerührt und dann bei 35° eine Lösung von 13,4 g (0,034 Mol) 4-Cyano-1-(2′-hydroxy-1′-tetradecyl)-4-phenylpiperidin (Beispiel 1e) in 160 ml abs. Tetrahydrofuran zugetropft. Das Reaktionsgemisch wird anschliessend während 3 Stunden bei ca. 50° gerührt. Nach dem Abkühlen (0°) setzt man vorsichtig 120 ml konz. Natronlauge zu, bis das Gemisch alkalisch reagiert. Die Emulsion wird durch Kieselgur (Hyflo Super Cel, Fluka) filtriert, das Filtrat mit Ether extrahiert, die organische Phase über Natriumsulfat getrocknet und eingedampft. Der Rückstand wird in wenig Ethanol gelöst und mit einem kleinen Ueberschuss ethanolischer Salzsäure vesetzt. Die Titelverbindung kristallisiert dabei aus. Sie wird abfiltriert und getrocknet. Smp. 238-243°; freie Base Smp. 71-72°.

(f) R-(-)4-Aminomethyl-1-(2′-hydroxy-1′-tetradecyl)-4-phenylpiperidin dihydrochlorid, Smp. 240-243° (unter Braunfärbung); freie Base, Smp. 65-66°.
(g) 4-Aminomethyl-1-(2′-hydroxy-1′-hexadecyl)-4-phenylpiperidin-dihydrochlorid, Smp. 243-245°; frei Base, Smp. 76-77°.
(h) 4-Aminomethyl-1-(2′-hydroxy-1′-octadecyl)-4-phenylpiperidin-dihydrochlorid, Smp. 252-254°.
(i) 4-Aminomethyl-1-(2′-hydroxy-1′-butyl)-4-phenylpiperidin-dihydrochlorid, Smp. 265-267°.

Beispiel 3: (Zwei Diastereomere von) 4-Cyano-1-[2′-(1″-phenylethylaminocarbonyloxy)-1′-tetradecyl]-4-phenylpiperidin

2.0 g (0.005 Mol) 4-Cyano-1-(2′-hydroxy-1′-tetradecyl)-4-phenylpiperidin (Beispiel 1e) werden in 5 ml Methylenchlorid gelöst und bei Raumtemperatur mit 1.0 ml (0.0071 Mol) (-)-1-Phenylethylisocyanat (Fluka) versetzt. Nach beendeter Reaktion (ca. 16 h) wird die Reaktionslösung eingedampft. Durch zweimalige Umkristallisation aus Methanol kann ein (-)-drehendes 4-Cyano-1-[2′-(1″-phenylethylaminocarbonyloxy)-1′-tetradecyl]-4-phenylpiperidin (Isomer A) rein erhalten werden, Smp. 59-63°, $[\alpha]_D^{20}$ = -46.3° ± 0.9° (c=1 in Ethanol). Durch Chromatographie der Mutterlaugen an Kieselgel 60 (Eluiermittel: Petrolether/Essigester 4:1) kann auch das zweite ebenfalls (-)-drehende 4-Cyano-1-[2′-(1″-phenylethylaminocarbonyloxy)-1′-tetradecyl]-4-phenylpiperidin (Isomer B) als Oel erhalten werden, $[\alpha]_D^{20}$ = -21.1° (c=1 in Ethanol). Unter den angegebenen chromatographischen Bedingungen eluiert zuerst das Diastereomere A.

Beispiel 4: (a) S-(+)-4-Aminomethyl-1-(2′-hydroxy-1′-tetradecyl)-4-phenylpiperidin-dihydrochlorid

Eine Lösung von 19.1 g (0.143 Mol) Aluminiumtrichlorid in 300 ml abs. THF wird bei Raumtemperatur und unter Stickstoff in eine Suspension von 5.67 g (0.143 Mol) Lithiumaluminiumhydrid in 390 ml abs. THF getropft. Nach 15 min fügt man eine Lösung von 20.3 g (0.0372 Mol) 4-Cyano-1-[2′-(1″-phenylethylaminocarbonyloxy)-1′-tetradecyl]-4-phenylpiperidin (Isomer B, s. Beispiel 3) in 390 ml abs. THF tropfenweise zu. Das Reaktionsgemisch wird 3.5 h bei 40 bis 50° gerührt, abgekühlt und durch Zugabe von 400 ml einer 30 %igen Natriumhydroxydlösung hydrolisiert (kühlen im Eisbad). Danach wird mit 800 ml Ether und 200 ml Wasser verdünnt und im Scheidetrichter gut geschüttelt. Die wässrige Phase extrahiert man mit Ether, wäscht die organische Phase mit gesättigter Natriumchloridlösung, trocknet sie über Natriumsulfat und dampft ein. Der Rückstand wird in 20 ml Ethanol gelöst und mit 50 ml einer gesättigten ethanolischen Salzsäurelösung versetzt. Beim Abkühlen kristallisiert das Produkt aus. Man filtriert, wäscht mit kaltem Ethanol und trocknet am Hochvakuum; Smp. 234-238° (unter Braunfärbung), $[\alpha]_D^{20}$ = +13.0° ± 0.9° (c=1 in Methanol).

(b) In analoger Weise wird aus Isomer A (s. Beispiel 3) R-(-)-4-Aminomethyl-1-(2′-hydroxy-1′-tetradecyl)-4-phenylpiperidin-dihydrochlorid hergestellt, Smp. 236-239° (unter Braunfärbung), $[\alpha]_D^{20}$ = -13.6° ± 0.9° (c=1.1 in Methanol).

Beispiel 5: 4-Cyano-4-phenyl-1-tetradecylpiperidin

11 g (0.05 Mol) 4-Cyano-4-phenylpiperidin-hydrochlorid (Aldrich), 41 g (0.3 Mol) Kaliumcarbonat (gemahlen, wasserfrei) und 13 ml (0.05 Mol) Tetradecylbromid werden in 150 ml Acetonitril während 5 h unter Rückfluss erhitzt. Nach dem Abkühlen wird von den unlöslichen anorganischen Salzen abfiltriert, das Filtrat eingedampft, der Rückstand in Methylenchlorid aufgenommen und mit Wasser und gesättigter Natriumchloridlösung gewaschen. Man trocknet die organische Phase über Magnesiumsulfat, dampft ein und kristallisiert den Rückstand aus Methanol um; Smp. 46-48°.

Beispiel 6: 4-Aminomethyl-4-phenyl-1-tetradecylpiperidin

Eine Lösung von 11.9 g (0.089 Mol) Aluminiumtrichlorid in 175 ml abs. THF wird unter Stickstoff und bei Raumtemperatur in eine Suspension von 3.4 g (0.089 Mol) Lithiumaluminiumhydrid in 220 ml abs. THF getropft. Es wird 15 min gerührt und dann auf 35° erwärmt. Zu dieser Mischung tropft man eine Lösung von 14.5 g (0.089 Mol) 4-Cyano-4-phenyl-1-tetradecylpiperidin (s. Beispiel 5) in 175 ml abs. THF zu. Das

Reaktionsgemisch wird anschliessend 3 h bei 40-50° gerührt. Nach dem Abkühlen auf Raumtemperatur setzt man vorsichtig ca. 130 ml konz. Natronlauge zu, bis das Gemisch alkalisch reagiert. Die Emulsion wird durch Kieselgur (Hyflo Super Cel, Fluka) filtriert, das Filtrat mit Ether extrahiert, die organische Phase über Natriumsulfat getrocknet und eingedampft. Der resultierende Rückstand wird aus 95 %igem Methanol umkristallisiert; Smp. 44-46°.

Beispiel 7: 1-Tetradecyl-4-piperidincarbonsäureamid

2.5 g (0.02 Mol) 4-Piperidincarbonsäureamid (Jansen) werden in 60 ml Acetonitril gelöst und nach Zugabe von 8.2 g (0.06 Mol) Kaliumcarbonat (gemahlen, wasserfrei) und 5.4 ml (0.02 Mol) Tetradecylbromid während 22 h unter Rückfluss gekocht, abgekühlt und filtriert. Der Feststoff wird mit einem Gemisch aus Methylenchlorid/Ethanol (9:1) extrahiert; man dampft anschliessend das Filtrat und die Extrakte ein und kristallisiert aus Ethanol um; Smp. 120-125°.

Beispiel 8: 4-Aminomethyl-1-tetradecylpiperidin-dihydrochlorid

Zu einer Suspension von 1.7 g (0.045 Mol) Lithiumaluminiumhydrid in 50 ml abs. THF tropft man unter Stickstoff und bei Raumtemperatur eine warme, gesättigte Lösung von 3.2 g (0.01 Mol) 1-Tetradecyl-4-piperidincarbonsäureamid (s. Beispiel 7) in 350 ml abs. THF zu. Danach erhitzt man das Reaktionsgemisch 7 h unter Rückfluss, kühlt ab und versetzt vorsichtig mit 1.1 ml Wasser, 1.1 ml 15 %iger Natronlauge und 3.3 ml Wasser. Nach dem Filtrieren werden die anorganischen Salze gut mit Ether gewaschen, die Etherphase über Natriumsulfat getrocknet und eingedampft. Das resultierende Oel löst man in wenig Ethanol und versetzt es mit 2 Aequivalenten einer 10 %igen ethanolischen Salzsäurelösung. Dabei fällt das Produkt aus. Es wird abfiltriert und getrocknet; Smp. 240° (Zersetzung).

Beispiel 9: 1-(2′-Hydroxy-1′-tetradecyl)-4-piperidincarbonsäureamid

2.56 g (0.02 Mol) 1,2-Epoxytetradecan (Aldrich), 4.24 g (0.02 Mol) 4-Piperidincarbonsäureamid (Jansen) und 25 ml Ethanol werden 2 h unter Rückfluss erhitzt. Danach kühlt man ab, gibt 10 ml Ether zu und filtriert das Produkt ab; Smp. 135-137°.

Beispiel 10: 4-Aminomethyl-1-(2′-hydroxy-1′-tetradecyl)-piperidin-dihydrochlorid

Zu einer Suspension von 1.1 g (0.029 Mol) Lithiumaluminiumhydrid in 50 ml abs. THF tropft man unter Stickstoff und bei Raumtemperatur eine Lösung von 3.4 g (0.01 Mol) 1-(2′-Hydroxy-1′-tetradecyl)-4-piperidincarbonsäureamid (Beispiel 9) in 80 ml abs. THF zu. Danach erhitzt man das Reaktionsgemisch 4 h unter Rückfluss, kühlt ab, versetzt vorsichtig mit 1.1 ml Wasser, 1.1 ml 15 %iger Natronlauge und 3.3 ml Wasser und filtriert. Die anorganischen Salze werden gut mit Ether gewaschen, die vereinigten organischen Phasen über Natriumsulfat getrocknet und eingedampft. Das resultierende Oel löst man in wenig Ethanol und versetzt es mit 2 Aequivalenten einer 10 %igen ethanolischen Salzsäurelösung. Dabei fällt das Produkt aus. Es wird abfiltriert und getrocknet; Smp. 280°.

Beispiel 11: 1-(2′-Hydroxy-1′-tetradecyl)-piperidin-4-carbonsäureethylester

4.2 g (0.02 Mol) 1,2-Epoxytetradecan und 3.1 g (0.02 Mol) Piperidin-4-carbonsäureethylester werden zusammen in 20 ml Ethanol während 5.5 h unter Rückfluss erhitzt. Die Reaktionslösung wird anschliessend abgekühlt, eingedampft und der Rückstand aus wässrigem Ethanol umkristallisiert; Smp. 33-34°.

Beispiel 12: 1-(2′-Hydroxy-1′-tetradecyl)-piperidin-4-carbonsäure

18.5 g (0.05 Mol) 1-(2′-Hydroxy-1′-tetradecyl)-piperidin-4-carbonsäureethylester (Beispiel 11) werden in 100 ml Ethanol gelöst und nach Zugabe von 50 ml 2 N Natriumhydroxydlösung während 2 h unter

Rückfluss erhitzt. Die Reaktionslösung wird anschliessend bei 0° mittels 2N Salzsäure (50 ml ≘ 0.1 Mol) neutralisiert und so weit wie möglich eingeengt. Der Rückstand wird mit 400 ml Methylenchlorid verrührt, über Natriumsulfat getrocknet und eingedampft. Der kristalline Rückstand wird mit Ether/Petrolether (1:1) verrieben, filtriert und getrocknet; Smp. 140-145°.

Beispiel 13: 4-Aminomethyl-1-(2′-hydroxy-1′-tetradecyl)-4-methylpiperidin-dihydrochlorid

0.99 g (0.002 Mol) 4-Aminomethyl-1-(2′-thexyldimethylsilyloxy-1′-tetradecyl)-4-methylpiperidin-hydrochlorid werden in einer 3 %igen Lösung von konz. Salzsäure in Ethanol gelöst und während 7 h unter Rückfluss erhitzt. Die erkaltete Reaktionslösung wird eingedampft, man nimmt den Rückstand in 50 ml Methylenchlorid auf, wäscht mit gesättigter Natriumcarbonatlösung, trocknet über Natriumsulfat und dampft erneut ein. Das so erhaltene Oel wird in wenig Ethanol gelöst, mit 1.5 ml einer 10 %igen ethanolischen Salzsäurelösung versetzt, etwas aufkonzentriert und durch Zugabe von wenig Ether zur Kristallisation gebracht. Die Titelverbindung zersetzt sich bei ca. 260°.

Die Ausgangsverbindung wird wie folgt hergestellt:

(a) 1-(2′-Thexyldimethylsilyloxy-1′-tetradecyl)-piperidin-4-carbonsäureethylester: 1.84 g (0.005 Mol) 1-(2′-Hydroxy-1′-tetradecyl)-piperidin-4-carbonsäureethylester (Beispiel 11) und 0.34 g (0.005 Mol) Imidazol werden in 10 ml Dimethylformamid gelöst und bei Raumtemperatur mit 0.89 ml (0.005 Mol) Thexyldimethylchlorsilan (Fluka) [Thexyl ≘ 2,3-Dimethyl-2-butyl) versetzt und 24 h bei Raumtemperatur gerührt. Man dampft ein, extrahiert den Rückstand mit einem Gemisch von Ether/Petrolether 1:1, wäscht mit gesättigter Natriumbicarbonatlösung ($NaHCO_3$), trocknet über Natriumsulfat und dampft ein. Der Rückstand wird chromatographiert (Kieselgel 60, Essigester/Petrolether 2:1). Die Titelverbindung eluiert mit den ersten Fraktionen (Rf-Wert = 0.88); IR: 1709 $cm^{-1}$ (C=0), Analyse: gefunden: C 69.9 % H 12.2 % N 2.5 %, berechnet: C 70.4 % H 11.9 % N 2.7 %.

(b) 1-(2′-Thexyldimethylsilyloxy-1′-tetradecyl)-4-methylpiperidin-4-carbonsäureethylester: Eine Lösung von 18.2 g (0.035 Mol) 1-(2′-Thexyldimethylsilyloxy-1′-tetradecyl)-piperidin-4-carbonsäureethylester in 50 ml abs. THF wird bei -78° und unter Stickstoff zu einer Lösung von Lithiumdiisopropylamid in 60 ml abs. THF [hergestellt aus 7.3 ml (0.052 Mol) Diisopropylamin und 32.4 ml (0.052 Mol) einer 1.6 molaren Butyllithiumlösung in Hexan] getropft. Nach 1 h bei -78° werden 2.2 ml (0. 035 Mol) Methyliodid zugetropft. Man lässt 2 h bei -78° rühren, lässt das Gemisch langsam auf Raumtemperatur erwärmen und rührt über Nacht aus. Durch tropfenweise Zugabe von 5.5 ml 50 %iger Essigsäure bei 0° wird das Reaktionsgemisch hydrolisiert. Dann verdünnt man mit Ether, wäscht mit gesättigter Natriumbicarbonatlösung, trocknet über Natriumsulfat und destilliert das Lösungsmittel im Rotationsverdampfer ab. Chromatographie an Kieselgel 60 (Essigester/Petrolether 1:9) liefert die Titelverbindung als Oel (Rf-Wert = 0.44 in obigem System).

(c) 1-(2′-Thexyldimethylsilyloxy-1′-tetradecyl)-4-methylpiperidin-4-carbonsäure: 15.6 g (0.03 Mol) 1-(2′-Thexyldimethylsilyloxy-1′-tetradecyl)-4-methylpiperidin-4-carbonsäureethylester werden in 180 ml Ethanol gelöst und mit 30 ml 2N Natriumhydroxydlösung behandelt. Man erhitzt 5 h unter Rückfluss, kühlt ab und neutralisiert mit 30 ml 2 N Salzsäure. Der Alkohol wird abdestilliert, der Rückstand zwischen Methylenchlorid und Wasser verteilt, die organische Phase über Natriumsulfat getrocknet, eingedampft und am Hochvakuum getrocknet. Die so erhaltene Titelverbindung gelangt ohne weitere Reinigung in die nächste Synthesestufe.

(d) 4-Methyl-1-(2′-thexyldimethylsilyloxy-1′-tetradecyl)-piperidin-4-carbonsäureamid: 21 g (0.042 Mol) 1-(2′-Thexyldimethylsilyloxy-1′-tetradecyl)-4-methylpiperidin-4-carbonsäure werden in 590 ml abs. THF gelöst, auf 0° abgekühlt und mit 43.7 ml Pyridin und anschliessend tropfenweise mit 43.7 ml (0.355 Mol) Chlorameisensäureethylester versetzt. Dann werden sofort 135 ml konz. Ammoniaklösung zugetropft und das Gemisch mit 680 ml Wasser verdünnt. Man extrahiert mit Essigester, wäscht die organische Phase mit gesättigter Natriumbicarbonatlösung ($NaHCO_3$), trocknet über Natriumsulfat und dampft ein. Zur Reinigung wird an Kieselgel 60 (Methylenchlorid/Ethanol 4:1) chromatographiert (Rf-Wert in diesem System = 0.52). Die Titelverbindung wird als Oel erhalten und ohne weitere Reinigung in der nächsten Stufe eingesetzt.

(e) 9.69 g (0.0195 Mol) 4-Methyl-1-(2′-thexyldimethylsilyloxy-1′-tetradecyl)-piperidin-4-carbonsäureamid werden in 115 ml abs. THF gelöst und bei Raumtemperatur und unter Stickstoff zu einer Suspension von 1.5 g (0.0388 Mol) Lithiumaluminiumhydrid in 115 ml abs. THF getropft. Die Mischung wird anschliessend 2 h unter Rückfluss erhitzt, danach auf 0° abgekühlt und durch Zugabe von 1.5 ml Wasser, 1.5 ml einer 15 %igen Natriumhydroxydlösung und 4.5 ml Wasser hydrolisiert. Man verdünnt das Gemisch mit 200 ml Ether, filtriert, wäscht das Filtrat mit Wasser, trocknet es über Natriumsulfat und dampft es ein. Das Produkt wird durch Chromatographie an Kieselgel 60 (Chloroform/Methanol/konzentrierte Ammoniaklösung 150:50:1) gereinigt und durch Behandlung mit einer 10 %igen ethanolischen Salzsäurelösung in das

Dihydrochlorid überführt. Man erhält das 4-Aminomethyl-1-(2′-thexyldimethylsilyloxy-1′-tetradecyl)-4-methylpiperidin-dihydrochlorid, welches wachsartig ist und bei 80° schmilzt.

Beispiel 14: 1-(2′-Hydroxy-1′-tetradecyl)-4-methylpiperidin-4-carbonsäureethylester

8.3 g (0.015 Mol) 1-(2′-Thexyldimethylsilyloxy-1′-tetradecyl)-4-methylpiperidin-4-carbonsäureethylester (Beispiel 13b) werden in 120 ml 1 %iger konz. Salzsäure in Ethanol gelöst und während 12 h unter Rückfluss erhitzt. Nach dem Abkühlen dampft man ein, nimmt den Rückstand in Methylenchlorid auf, wäscht mit gesättigter Natriumbicarbonatlösung, trocknet über Natriumsulfat und dampft erneut ein. Das resultierende Oel wird an Kieselgel 60 chromatographiert (Essigester/Petrolether 8:2). Die reinen Fraktionen ergeben die Titelverbindung als Oel; Massenspektrum: 383, 184.

Beispiel 15: 1-(2′-Hydroxy-1′-tetradecyl)-4-methylpiperidin-4-carbonsäure

2 g (0.005 Mol) 1-(2′-Hydroxy-1′-tetradecyl)-4-methylpiperidin-4-carbonsäureethylester (Beispiel 14) werden in 30 ml Ethanol gelöst und nach Zugabe von 5 ml (0.01 Mol) 2 N Natriumhydroxydlösung 4 h unter Rückfluss erwärmt. Zur Aufarbeitung wird auf 0° abgekühlt, mit 5 ml (0.01 Mol) 2 N Salzsäure neutralisiert und am Rotationsverdampfer der Alkohol abdestilliert. Der wässrige Rückstand wird mit Methylenchlorid extrahiert, die organische Phase mit Wasser gewaschen, über Natriumsulfat getrocknet und eingedampft. Der feste Rückstand wird aus Methanol umkristallisiert. Die Titelverbindung schmilzt bei 198-200° Massenspektrum: 355, 156.

Beispiel 16: 4-Hydroxymethyl-1-(2′-hydroxy-1′-tetradecyl)-4-methylpiperidin-hydrochlorid

0.15 g (0.004 Mol) Lithiumaluminiumhydrid werden unter Stickstoff in 15 ml abs. Ether vorgelegt. Man tropft eine Lösung von 0.8 g (0.002 Mol) 1-(2′-Hydroxy-1′-tetradecyl)-4-methylpiperidin-4-carbonsäureethylester (Beispiel 14) zu und rührt 12 h nach. Das Reaktionsgemisch wird der Reihe nach mit 0.15 ml Wasser, 0.15 ml 15 %iger Natronlauge und 0.45 ml Wasser versetzt, etwas mit Ether verdünnt und filtriert. Das Filtrat wird über Natriumsulfat getrocknet und eingedampft. Der Rückstand wird mittels 10 %iger ethanolischer Salzsäure ins Hydrochlorid überführt; Smp. 100° (wachsartige Verbindung).

Beispiel 17: 4-Hydroxymethyl-1-(2′-hydroxy-1′-tetradecyl)-piperidin-hydrochlorid

0.76 g (0.02 Mol) Lithiumaluminiumhydrid werden unter Stickstoff in 50 ml abs. Ether vorgelegt. Man tropft eine Lösung von 3.69 g (0.01 Mol) 1-(2′-Hydroxy-1′-tetradecyl)-piperidin-4-carbonsäureethylester (60 ml Ether) (Beispiel 11) zu und rührt 1.5 h nach. Das Reaktionsgemisch wird der Reihe nach mit 0.75 ml Wasser, 0.75 ml 15 %iger Natronlauge und 2.28 ml Wasser versetzt, etwas mit Ether verdünnt und filtriert. Das Filtrat wird über Natriumsulfat getrocknet und eingedampft. Der Rückstand wird aus Petrolether umkristallisiert (Smp. der freien Base 40-42°). Das Hydrochlorid erhält man durch Lösen der freien Base in wenig Ethanol, Zugabe von 10 %iger ethanolischer Salzsäurelösung und Aufkonzentrierung; Smp. 110°.

Beispiel 18: 4-Cyano-1-(2′-decanoyloxy-1′-tetradecyl)-4-phenylpiperidin-hydrochlorid

15.92 g (0.04 Mol) 4-Cyano-1-(2′-hydroxy-1′-tetradecyl)-4-phenylpiperidin (Beispiel 1e) werden in 40 ml Methylenchlorid vorgelegt und bei Raumtemperatur tropfenweise mit 11.4 g (0.06 Mol) Decanoylchlorid behandelt. Das Reaktionsgemisch wird 15 min bei Raumtemperatur gerührt, dann 30 min unter Rückfluss erwärmt. Nach dem Abkühlen wird mit gesättigter Natriumbicarbonatlösung gewaschen, über Natriumsulfat getrocknet und einge dampft. Der Rückstand wird durch Chromatographie an Kieselgel 60 (Essigester/Petrolether 3:7) gereinigt. Das Hydrochlorid wird durch Behandlung der reinen Fraktionen mit ethanolischer Salzsäure erhalten; Smp. 63-65°.

Beispiel 19: 4-Aminomethyl-1-(2′-decanoyloxy-1′-tetradecyl)-4-phenylpiperidin-dihydrochlorid

33.6 g (0.06 Mol) 4-Cyano-1-(2′-decanoyloxy-1′-tetradecyl)-4-phenylpiperidin (Beispiel 18) werden in 500 ml Essigsäure und mit 3.6 g Platinoxyd bei Normaldruck hydriert. Nach 14 h kommt die Reaktion zum Stillstand, man filtriert den Katalysator ab und dampft das Filtrat ein. Der Rückstand wird in Methylenchlorid aufgenommen, mit gesättigter Natriumbicarbonatlösung gewaschen, über Natriumsulfat getrocknet und erneut eingedampft. Chromatographie an Kieselgel 60 mittels Methylenchlorid/Methanol (7:3) liefert die freie Base der Titelverbindung (Oel), welche in Ethanol gelöst und durch Zugabe von 10 %iger ethanolischer Salzsäure in das Hydrochlorid überführt wird; Smp. 85-87°.

Beispiel 20: 4-Hydroxymethyl-1-(3′-hydroxy-1′-tetradecyl)-piperidin

Zu einer unter Feuchtigkeitsausschluss gerührten Suspension von 0.25 g (6.6 mMol) Lithiumaluminiumhydrid in 10 ml abs. THF wird bei 50° tropfenweise eine Lösung von 1.2 g (3.1 mMol) 1-(3′-Hydroxymyristoyl)-piperidin-4-carbonsäureethylester in 20 ml abs. THF zugegeben. Man rührt 16 h bei dieser Temperatur. Nach dem Abkühlen werden vorsichtig 10 ml 5 N Natronlauge und 20 ml Wasser zugesetzt, und man extrahiert zweimal mit 50 ml Ether. Die Etherlösungen werden mit Wasser gewaschen, über Natriumsulfat getrocknet und eingedampft. Man erhält so die Titelverbindung als gelbliches Oel, Rf-Wert = 0,4 (Kieselgeldünnschichtplatten, Methylenchlorid : Methanol : konz. Ammoniak 40:20:1).

Die Ausgangsverbindung wird wie folgt hergestellt:

Eine Lösung von 2.4 g (10 mMol) 3-Hydroxymyristinsäure in 100 ml Dimethylformamid wird mit 1.57 g (10 mMol) Piperidin-4-carbonsäureethylester und 5.0 g (20 mMol) 2-Ethoxy-1-ethoxycarbonyl-1,2-dihydrochinolin (EEDQ) versetzt und 30 h bei 50° gerührt. Das Reaktionsgemisch wird eingedampft und der Rückstand mit Methylenchlorid/Essigester 1:1 über Kieselgel chromatographiert. Man erhält so den 1-(3′-Hydroxymyristoyl)-piperidin-4-carbonsäureethylester als gelbliches Oel, Rf-Wert = 0,32 (Kieselgeldünnschichtplatten, Methylenchlorid : Essigester 1:1).

Beispiel 21: 4-Aminomethyl-1-(3′-hydroxy-1′-tetradecyl)-4-phenylpiperidin-Oxalat

Eine unter Feuchtigkeitsausschluss gerührte Suspension von 0.175 g (4.6 mMol) Lithiumaluminiumhydrid in 5 ml THF wird mit einer Lösung von 0.613 g (4.6 mMol) Aluminiumtrichlorid in 5 ml THF versetzt und auf 50° erwärmt. Zu diesem Gemisch wird eine Lösung von 0.76 g (1.84 mMol) 4-Cyano-1-(3′-hydroxymyristoyl)-4-phenylpiperidin in 10 ml THF tropfenweise zugegeben, und man rührt 16 h nach. Das Reaktionsgemisch wird analog Beispiel 20 aufgearbeitet und das erhaltene Produkt als Oxalat aus Methanol kristallisiert; Smp. 190-192°.

Die Ausgangsverbindung wird wie folgt hergestellt:

Eine Lösung von 2.4 g (10 mMol) 3-Hydroxymyristinsäure in 100 ml Dimethylformamid wird nacheinander mit 1.38 ml (10 mMol) Triethylamin, 2.3 g (10 mMol) 4-Cyano-4-phenylpiperidin-hydrochlorid und 5.0 g (20 mMol) 2-Ethoxy-1-ethoxycarbonyl-1,2-dihydrochinolin (EEDQ) versetzt und 30 h bei 50° gerührt. Das Reaktionsgemisch wird eingedampft und der Rückstand über 250 g Kieselgel mit Methylenchlorid/Essigester (1:1) chromatographiert. Man erhält so das 4-Cyano-1-(3′-hydroxymyristoyl)-4-phenylpiperidin als gelbes Oel.

Beispiel 22: 4-(N-Acetylaminomethyl)-1-(2′-hydroxy-1′-dodecyl)-4-phenylpiperidin-hydrochlorid

2 g (0.0053 Mol) 4-Aminomethyl-1-(2′-hydroxy-1′-dodecyl)-4-phenylpiperidin (Beispiel 2d), 10 ml Pyridin und 10 ml Acetanhydrid werden 16 h bei Raumtemperatur gerührt. Nach dem Abkühlen verdünnt man mit 150 ml Eiswasser und lässt 45 min stehen. Nach Extraktion mit Essigester wird die organische Phase mit 2 N Salzsäure, 2 N Natriumhydroxydlösung und gesättigter Natriumchloridlösung gewaschen, über Natriumsulfat getrocknet und eingedampft. Der Rückstand wird an Kieselgel 60 chromatographiert (Gradient: Toluol-Essigester 80:20 bis 40:60). Die Diacetylverbindung enthaltenden Fraktionen werden eingedampft, der Rückstand in 32 ml Methanol gelöst und mit 1.21 g (0.00875 Mol) Kaliumcarbonat in 8 ml Wasser versetzt. Nach 17 h Rühren bei Raumtemperatur wird die gelbe Lösung mit 100 ml einer gesättigten Natriumchloridlösung versetzt und mit Essigester extrahiert. Die organische Phase wird mit gesättigter Natriumchloridlösung gewaschen, über Natriumsulfat getrocknet und eingedampft. Chromatographie des Rückstandes an Kieselgel 60 (Gradient: reines Chloroform bis Chloroform-Methanol 95:5) und Umkristallisation der reinen Fraktionen aus Methylenchlorid/Ether/Petrolether liefert die Titelverbindung als

freie Base (Smp. 68-70°). Das Hydrochlorid erhält man durch Zusatz von ethanolischer Salzsäure und Kristallisation aus Ether; Smp. 70-75°.

Beispiel 23:

Kapseln enthaltend 0,25 g Wirkstoff, z.B. eine der Verbindungen der Beispiele 1-22, können wie folgt hergestellt werden:

| Zusammensetzung (für 5000 Kapseln) | |
|---|---|
| Wirkstoff | 1250 g |
| Talk | 180 g |
| Weizenstärke | 120 g |
| Magnesiumstearat | 80 g |
| Laktose | 20 g |

Die pulverförmigen Substanzen werden durch ein Sieb mit einer Maschenweite von 0,6 mm getrieben und gemischt. Portionen von je 0,33 g des Gemisches werden mittels einer Kapselfüllmaschine in Gelatine-Kapseln abgefüllt.

## Ansprüche

1. Verbindungen der Formel I

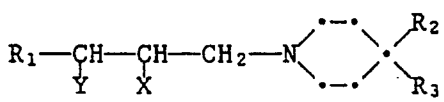

(I)

worin $R_1$ $C_1$-$C_{30}$-Alkyl bedeutet; $R_2$ Carboxy, Niederalkoxycarbonyl, Carbamoyl, N-Niederalkylcarbamoyl, N,N-Diniederalkylcarbamoyl, Cyano oder Niederalkyl, welches durch Hydroxy, Niederalkoxy, Acyloxy, Amino, Niederalkylamino, Diniederalkylamino oder Acylamino substituiert ist, bedeutet; $R_3$ für Wasserstoff, Niederalkyl oder Aryl steht; worin einer der Reste X und Y Hydroxy, Niederalkoxy oder Acyloxy bedeutet und der andere der Reste X und Y für Wasserstoff steht; oder worin beide Reste X und Y auch Wasserstoff bedeuten können, wenn $R_2$ für Cyano oder für durch Amino oder Acylamino substituiertes Niederalkyl steht und $R_1$ $C_2$-$C_{30}$-Alkyl bedeutet; und ihre Salze.

2. Verbindungen der Formel I gemäss Anspruch 1, worin $R_1$ $C_1$-$C_{19}$-Alkyl bedeutet; $R_2$ Carboxy, Niederalkoxycarbonyl, Carbamoyl, N-Niederalkylcarbamoyl, N,N-Diniederalkylcarbamoyl, Cyano oder Niederalkyl, welches durch Hydroxy, Niederalkoxy, Niederalkanoyloxy, Amino, Niederalkylamino, Diniederalkylamino oder Niederalkanoylamino substituiert ist, bedeutet; $R_3$ für Wasserstoff, Niederalkyl oder Phenyl steht; worin einer der Reste X und Y Hydroxy, Niederalkoxy, $C_1$-$C_{18}$-Alkanoyloxy, Carbamoyloxy, N-Niederalkylcarbamoyloxy, N-Phenylniederalkylcarbamoyloxy oder N-Phenylcarbamoyloxy bedeutet und der andere der Reste X und Y für Wasserstoff steht, oder worin beide Reste X und Y auch Wasserstoff bedeuten können, wenn $R_2$ für Cyano oder für durch Amino oder Niederalkanoylamino substituiertes Niederalkyl steht und $R_1$ $C_2$-$C_{19}$-Alkyl bedeutet, wobei Phenylreste unsubstituiert oder durch Niederalkyl, Halogen-niederalkyl, Hydroxy, Niederalkoxy, Niederalkanoyloxy, Halogen und/oder Nitro substituiert sind; und ihre Salze.

3. Verbindungen der Formel I gemäss Anspruch 1, worin $R_1$ $C_1$-$C_{15}$-Alkyl bedeutet; $R_2$ Carboxy, Niederalkoxycarbonyl, Carbamoyl, Cyano oder Niederalkyl, welches durch Hydroxy, Amino oder Niederalkanoylamino substituiert ist, bedeutet; $R_3$ für Wasserstoff, Niederalkyl oder Phenyl steht; worin einer der Reste X und Y Hydroxy, $C_1$-$C_{12}$-Alkanoyloxy oder N-Phenylniederalkylcarbamoyloxy bedeutet und der andere der Reste X und Y für Wasserstoff steht, oder worin beide Reste X und Y auch Wasserstoff bedeuten können, wenn $R_2$ für Aminomethyl steht und $R_1$ $C_2$-$C_{15}$-Alkyl bedeutet; und ihre Salze.

4. Verbindungen der Formel I gemäss Anspruch 1, worin $R_1$ $C_7$-$C_{15}$-Alkyl bedeutet; $R_2$ Carboxy, Niederalkoxycarbonyl, Carbamoyl, Hydroxymethyl, Aminomethyl oder Niederalkanoylaminomethyl bedeutet;

$R_3$ für Wasserstoff, Methyl oder Phenyl steht; worin einer der Reste X und Y Hydroxy bedeutet und der andere der Reste X und Y für Wasserstoff steht, oder worin beide Reste X und Y auch Wasserstoff bedeuten können, wenn $R_2$ für Aminomethyl steht; und ihre Salze.

5. Verbindungen der Formel I gemäss Anspruch 1, worin $R_1$ $C_7$-$C_{15}$-Alkyl bedeutet, $R_2$ für Aminomethyl steht, $R_3$ Phenyl bedeutet, und (a) X Hydroxy bedeutet und Y für Wasserstoff steht, oder (b) X Wasserstoff bedeutet und Y für Hydroxy steht, oder (c) X und Y Wasserstoff bedeuten; und ihre Salze.

6. 4-Aminomethyl-1-(2′-hydroxy-1′-tetradecyl)-piperidin und pharmazeutisch verwendbare Salze davon gemäss Anspruch 1.

7. 4-Aminomethyl-4-phenyl-1-tetradecylpiperidin und pharmazeutisch verwendbare Salze davon gemäss Anspruch 1.

8. 4-Aminomethyl-1-(2′-hydroxy-1′-tetradecyl)-4-phenylpiperidin und pharmazeutisch verwendbare Salze davon gemäss Anspruch 1.

9. 4-Aminomethyl-1-tetradecylpiperidin und pharmazeutisch verwendbare Salze davon gemäss Anspruch 1.

10. Pharmazeutisches Präparat enthaltend eine Verbindung gemäss einem der Ansprüche 1 bis 9 und mindestens ein pharmazeutisch verwendbares Trägermaterial.

11. Eine Verbindung gemäss einem der Ansprüche 1 bis 9 zur Anwendung in einem Verfahren zur therapeutischen Behandlung des tierischen oder menschlichen Körpers.

12. Eine Verbindung gemäss einem der Ansprüche 1 bis 9 zur Verwendung als antitumor-wirksames Mittel.

13. Verwendung von einer Verbindung gemäss einem der Ansprüche 1 bis 9 zur Herstellung pharmazeutischer Präparate.

14. Verfahren zur Herstellung einer Verbindung der Formel I gemäss Anspruch 1, dadurch gekennzeichnet, dass man

(a) eine Verbindung der Formel II

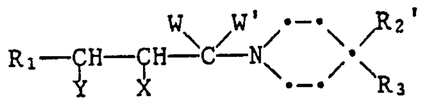

(II)

worin $R_1$, $R_3$, X und Y die unter Formel I angegebene Bedeutung haben, $R_2'$ eine Gruppe, die durch Reduktion in einen Rest $R_2$ überführbar ist, bedeutet, oder, falls W und W′ zusammen Oxo bedeuten, auch für einen Rest $R_2$ wie unter Formel I definiert stehen kann, und W und W′ zusammen eine Oxogruppe bedeuten oder für zwei Wasserstoffatome stehen, reduziert, oder

b) eine Verbindung der Formel III

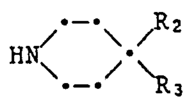

(III)

worin $R_2$ und $R_3$ die unter Formel I angegebene Bedeutung haben, mit einer Verbindung der Formel IV

$R_1$- CH(Y)- CH(X)-$CH_2$-Z (IV)

worin $R_1$, X und Y die unter Formel I angegebene Bedeutung haben und Z eine nukleofuge Abgangsgruppe ist und worin X und Z zusammen auch eine Epoxygruppe bedeuten können, alkyliert, oder

(c) zur Herstellung von Verbindungen der Formel I, worin X oder Y Hydroxy bedeutet, in einer Verbindung der Formel V

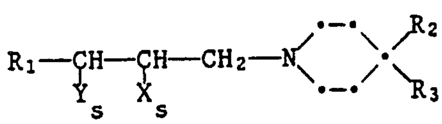

(V)

worin $R_1$, $R_2$ und $R_3$ die unter Formel I angegebene Bedeutung haben, einer der Reste $X_s$ und $Y_s$ eine durch eine Schutzgruppe geschützte Hydroxygruppe bedeutet und der andere der Reste $X_s$ und $Y_s$ für Wasserstoff steht, die Hydroxy-Schutzgruppe abspaltet; und/oder, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder ein erhaltenes Gemisch von isomeren Verbindungen der Formel I in die einzelnen Isomeren auftrennt.

15. Die nach den Verfahren gemäss Anspruch 14 erhältlichen Verbindungen.

Patentansprüche für folgende Vertragsstaaten: ES, GR

1. Verfahren zur Herstellung einer Verbindung der Formel I

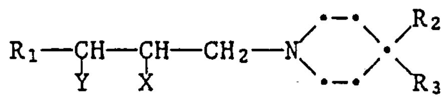

(I)

worin $R_1$ $C_1$-$C_{30}$-Alkyl bedeutet; $R_2$ Carboxy, Niederalkoxycarbonyl, Carbamoyl, N-Niederalkylcarbamoyl, N,N-Diniederalkylcarbamoyl, Cyano oder Niederalkyl, welches durch Hydroxy, Niederalkoxy, Acyloxy, Amino, Niederalkylamino, Diniederalkylamino oder Acylamino substituiert ist, bedeutet; $R_3$ für Wasserstoff, Niederalkyl oder Aryl steht; worin einer der Reste X und Y Hydroxy, Niederalkoxy oder Acyloxy bedeutet und der andere der Reste X und Y für Wasserstoff steht; oder worin beide Reste X und Y auch Wasserstoff bedeuten können, wenn $R_2$ für Cyano oder für durch Amino oder Acylamino substituiertes Niederalkyl steht und $R_1$ $C_2$-$C_{30}$-Alkyl bedeutet; und ihrer Salze, dadurch gekennzeichnet, dass man

(a) eine Verbindung der Formel II

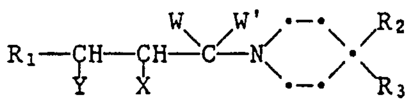

(II)

worin $R_1$, $R_3$, X und Y die unter Formel I angegebene Bedeutung haben, $R_2'$ eine Gruppe, die durch Reduktion in einen Rest $R_2$ überführbar ist, bedeutet, oder, falls W und W' zusammen Oxo bedeuten, auch für einen Rest $R_2$ wie unter Formel I definiert stehen kann, und W und W' zusammen eine Oxogruppe bedeuten oder für zwei Wasserstoffatome stehen, reduziert, oder

b) eine Verbindung der Formel III

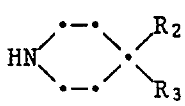

(III)

worin $R_2$ und $R_3$ die unter Formel I angegebene Bedeutung haben, mit einer Verbindung der Formel IV

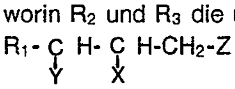 (IV)

worin $R_1$, X und Y die unter Formel I angegebene Bedeutung haben und Z eine nukleofuge Abgangsgruppe ist und worin X und Z zusammen auch eine Epoxygruppe bedeuten können, alkyliert, oder

(c) zur Herstellung von Verbindungen der Formel I, worin X oder Y Hydroxy bedeutet, in einer Verbindung der Formel V

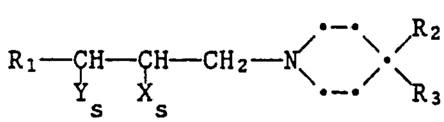

(V)

worin $R_1$, $R_2$ und $R_3$ die unter Formel I angegebene Bedeutung haben, einer der Reste $X_s$ und $Y_s$ eine durch eine Schutzgruppe geschützte Hydroxygruppe bedeutet und der andere der Reste $X_s$ und $Y_s$ für Wasserstoff steht, die Hydroxy-Schutzgruppe abspaltet; und/oder, wenn erwünscht, eine erhaltene Verbindung der Formel I in eine andere Verbindung der Formel I umwandelt, und/oder, wenn erwünscht, ein erhaltenes Salz in die freie Verbindung oder in ein anderes Salz umwandelt, und/oder, wenn erwünscht, eine erhaltene freie Verbindung der Formel I in ein Salz umwandelt, und/oder ein erhaltenes Gemisch von

isomeren Verbindungen der Formel I in die einzelnen Isomeren auftrennt.

2. Verfahren gemäss Anspruch 1 zur Herstellung einer Verbindung der Formel I, worin $R_1$ $C_1$-$C_{19}$-Alkyl bedeutet; $R_2$ Carboxy, Niederalkoxycarbonyl, Carbamoyl, N-Niederalkylcarbamoyl, N,N-Diniederalkylcarbamoyl, Cyano oder Niederalkyl, welches durch Hydroxy, Niederalkoxy, Niederalkanoyloxy, Amino, Niederalkylamino, Diniederalkylamino oder Niederalkanoylamino substituiert ist, bedeutet; $R_3$ für Wasserstoff, Niederalkyl oder Phenyl steht; worin einer der Reste X und Y Hydroxy, Niederalkoxy, $C_1$-$C_{18}$-Alkanoyloxy, Carbamoyloxy, N-Niederalkylcarbamoyloxy, N-Phenylniederalkylcarbamoyloxy oder N-Phenylcarbamoyloxy bedeutet und der andere der Reste X und Y für Wasserstoff steht, oder worin beide Reste X und Y auch Wasserstoff bedeuten können, wenn $R_2$ für Cyano oder für durch Amino oder Niederalkanoylamino substituiertes Niederalkyl steht und $R_1$ $C_2$-$C_{19}$-Alkyl bedeutet, wobei Phenylreste unsubstituiert oder durch Niederalkyl, Halogen-niederalkyl, Hydroxy, Niederalkoxy, Niederalkanoyloxy, Halogen und/oder Nitro substituiert sind; und ihrer Salze.

3. Verfahren gemäss Anspruch 1 zur Herstellung einer Verbindung der Formel I, worin $R_1$ $C_1$-$C_{15}$-Alkyl bedeutet; $R_2$ Carboxy, Niederalkoxycarbonyl, Carbamoyl, Cyano oder Niederalkyl, welches durch Hydroxy, Amino oder Niederalkanoylamino substituiert ist, bedeutet; $R_3$ für Wasserstoff, Niederalkyl oder Phenyl steht; worin einer der Reste X und Y Hydroxy, $C_1$-$C_{12}$-Alkanoyloxy oder N-Phenylniederalkylcarbamoyloxy bedeutet und der andere der Reste X und Y für Wasserstoff steht, oder worin beide Reste X und Y auch Wasserstoff bedeuten können, wenn $R_2$ für Aminomethyl steht und $R_1$ $C_2$-$C_{15}$-Alkyl bedeutet; und ihrer Salze.

4. Verfahren gemäss Anspruch 1 zur Herstellung einer Verbindung der Formel I, worin $R_1$ $C_7$-$C_{15}$-Alkyl bedeutet; $R_2$ Carboxy, Niederalkoxycarbonyl, Carbamoyl, Hydroxymethyl, Aminomethyl oder Niederalkanoylaminomethyl bedeutet; $R_3$ für Wasserstoff, Methyl oder Phenyl steht; worin einer der Reste X und Y Hydroxy bedeutet und der andere der Reste X und Y für Wasserstoff steht, oder worin beide Reste X und Y auch Wasserstoff bedeuten können, wenn $R_2$ für Aminomethyl steht; und ihrer Salze.

5. Verfahren gemäss Anspruch 1 zur Herstellung einer Verbindung der Formel I, worin $R_1$ $C_7$-$C_{15}$-Alkyl bedeutet, $R_2$ für Aminomethyl steht, $R_3$ Phenyl bedeutet, und (a) X Hydroxy bedeutet und Y für Wasserstoff steht, oder (b) X Wasserstoff bedeutet und Y für Hydroxy steht, oder (c) X und Y Wasserstoff bedeuten; und ihrer Salze.

6. Verfahren gemäss Anspruch 1 zur Herstellung von 4-Aminomethyl-1-(2′-hydroxy-1′-tetradecyl)-piperidin und pharmazeutisch verwendbarer Salze davon.

7. Verfahren gemäss Anspruch 1 zur Herstellung von 4-Aminomethyl-4-phenyl-1-tetradecylpiperidin und pharmazeutisch verwendbarer Salze davon.

8. Verfahren gemäss Anspruch 1 zur Herstellung von 4-Aminomethyl-1-(2′-hydroxy-1′-tetradecyl)-4-phenylpiperidin und pharmazeutisch verwendbarer Salze davon.

9. Verfahren gemäss Anspruch 1 zur Herstellung von 4-Aminomethyl-1-tetradecylpiperidin und pharmazeutisch verwendbarer Salze davon.